# EUROPEAN PATENT APPLICATION

(11) **EP 4 082 563 A1**
(43) Date of publication of application: **02.11.2022**
(21) Application number: 20906353.6
(22) Date of filing: 25.12.2020
(51) Int. Cl.: A61K 39/00, A61P 35/00, A61P 35/04, C07K 7/06, C07K 19/00, A61K 47/65, A61K 38/16

(54) **POST-OPERATION ADJUVANT THERAPY AGENT**

(30) Priority: 26.12.2019 JP 2019236947
(71) Applicant: Taiho Pharmaceutical Co., Ltd., Chiyoda-ku Tokyo 101-8444 (JP)
(72) Inventor: GOTO Risa, Tsukuba-shi, Ibaraki 300-2611 (JP)
(74) Representative: Altmann Stößel Dick Patentanwälte PartG mbB
(86) International application number: PCT/JP2020/048667
(87) International publication number: WO 2021/132550

(57) **Abstract**

The present invention provides an agent for adjuvant therapy of tumors that exerts remarkable inhibitory effects on tumor metastasis and recurrence while developing few side effects. The agent for adjuvant therapy comprises, as an active ingredient, a peptide having 4 linked CTL epitopes.

## Description

### Technical Field

The present invention relates to an agent for adjuvant therapy of tumors involving the use of a peptide having 4 linked CTL epitopes comprising 4 peptides capable of induction of HLA-A-restricted CTL responses.

### Background Art

Cancer is the top cause of death in Japan. Approximately 350,000 patients die of cancer every year, and cancer is still a serious disease nowadays. The treatment approaches for cancer that have been established are surgical resection, anti-cancer drug therapy, radiation therapy, and cancer immunotherapy. In case of early-stage cancer with a low degree of progression, a favorable outcome can be expected by definitive surgical treatment that removes all the cancer cells. In contrast, effects of definitive surgical treatment for cancer with a high degree of progression are limited, and the frequency of topical recurrence and metastasis occur is not low. While various factors can cause topical recurrence and metastasis, possible causes are considered: small cancer cells visually unobservable may remain unremoved by surgery, or some cancer cells might have already undergone hematogenous metastasis or lymphatic metastasis and microcarcinomas might have already started to grow in other organs.

In case of metastatic recurrence, in particular, cancer cells are considered to have spread throughout the body, and it is thus very difficult to completely remove cancer cells by surgical treatment. As therapeutic techniques for metastatic cancer, accordingly, various chemotherapy techniques, radiation therapy, and, in recent years, cancer immunotherapy have been established. Such therapeutic techniques have action mechanisms of killing cancer cells at molecular and cellular levels. In principle, accordingly, it is possible to completely cure cancer cells that have spread throughout the body. However, there are many cases where all therapeutic techniques become ineffective and may result in death, which is a serious issue of concern for cancer therapy. Accordingly, development of a novel therapeutic technique for recurrent and metastatic cancers is critical for cancer suppression.

From a different point of view, prevention of metastasis that may result in the serious outcome may improve the probability of success of definitive surgical treatment, and the outcome for a cancer patient may be improved to a significant extent. A therapy that is performed to prevent cancer recurrence or metastasis by eradicating cancer cells that are visually unobservable in surgery or minute metastatic cancer cells is referred to as an adjuvant therapy.

In Japan, for example, use of a tegafur-gimeracil-oteracil potassium combination drug is established as a technique of adjuvant therapy for Stage II (excluding T 1), IIIA, or IIIB gastric cancer after curative surgery, and a mortality risk for a group of patients subjected to administration of tegafur-gimeracil-oteracil potassium combination drugs is lower by 32% than that for a group of patients subjected only to surgical treatment (Non-Patent Document 1). For treatment of cancers, such as lung cancer, breast cancer, colon cancer, uterine cancer, and malignant melanoma, adjuvant therapy is also performed. As described above, however, there are many cases failing to prevent recurrence or metastasis by the adjuvant therapy. Accordingly, development of a novel agent for adjuvant therapy aimed at improving the treatment outcome is awaited.

In recent years, in addition to a post-operative chemotherapy as an adjuvant therapy, clinical trials that perform cancer immunotherapy as an adjuvant therapy are in progress. Cancer immunotherapy has an action mechanism of eradicating cancer cells by, for example, inducing, transplanting, or activating immune cells that can directly damage cancer cells, such as cytotoxic T lymphocytes (CTL) or natural killer (NK) cells. Thus, cancer immunotherapy can exert antitumor effects on visually unobservable microcarcinomas or cancer cells throughout the body as with chemotherapy.

One of methods of cancer immunotherapy; i.e., cancer peptide vaccine therapy, has been expected as a novel therapeutic technique for cancer for a long period of time. In cancer treatment using peptide vaccines, T cell receptors (TCR) on an epitope-specific cytotoxic T lymphocyte (CTL) recognizes a major histocompatibility complex (MHC) that presents an antigen peptide on a cancer cell surface and the CTL damages cancer cells. Thus, treatment effects are exerted.

Human MHC is referred to as the human leucocyte antigen (HLA) and various HLA types are known. Thus, development of a cancer peptide vaccine targeting a particular HLA type has been attempted. However, HLA types targeted by cancer vaccines are limited, and, disadvantageously, a cancer peptide vaccine is not beneficial for a patient with an untargeted HLA type. In addition, a need of HLA typing before initiation of treatment delays the initiation of treatment, and it increases patient burden. Thus, research and development of a cancer peptide vaccine that is applicable to all cancer patients without a need of HLA typing have been desired.

As a solution to the problem described above, the peptide having 4 linked CTL epitopes comprising various CTL epitope peptides ligated to each other that can induce CTL responses to one or more HLA types selected from among HLA-A2, HLA-A24, HLA-A26, and HLA-A3 supertype has been reported (Patent Document 1).

However, whether or not the peptide having 4 linked CTL epitopes would exert effects as an adjuvant therapy has not yet been demonstrated.

### Prior Art Documents

### Patent Documents

Patent Document 1: WO 2015/060235

### Non-Patent Documents

Non-Patent Document 1: N. Engl. J. Med., 357: 1810-20, 2007

### Summary of the Invention

### Objects to Be Attained by the Invention

The present invention provides an agent for adjuvant therapy of tumors that exerts remarkable inhibitory effects on tumor metastasis and recurrence and has few side effects.

### Means for Attaining the Objects

The present inventor administered the peptide having 4 linked CTL epitopes to animal models of tumor metastasis and recurrence and examined inhibitory effects on tumor metastasis and recurrence. As a result, the present inventor discovered that the number of metastatic pulmonary nodules can be reduced to a significant extent without causing any serious side effects.

Specifically, the present invention provides [1] to [11] described below.
[1] An agent for adjuvant therapy of tumors comprising, as an active ingredient, a peptide having 4 linked CTL epitopes comprising 4 CTL epitope peptides linked via linkers, wherein the peptide having 4 linked CTL epitopes comprises the epitope peptide "PEP5" as shown in SEQ ID NO: 5 and 3 epitope peptides selected from the group consisting of the epitope peptide "PEP1" as shown in SEQ ID NO: 1, the epitope peptide "PEP2" as shown in SEQ ID NO: 2, the epitope peptide "PEP4" as shown in SEQ ID NO: 4, the epitope peptide "PEP6" as shown in SEQ ID NO: 6, the epitope peptide "PEP7" as shown in SEQ ID NO: 7, the epitope peptide "PEP8" as shown in SEQ ID NO: 8, the epitope peptide "PEP9" as shown in SEQ ID NO: 9, the epitope peptide "PEP10" as shown in SEQ ID NO: 10, the epitope peptide "PEP13" as shown in SEQ ID NO: 13, the epitope peptide "PEP15" as shown in SEQ ID NO: 15, the epitope peptide "PEP17" as shown in SEQ ID NO: 17, and the epitope peptide "PEP18" as shown in SEQ ID NO: 18 linked via linkers, wherein the peptide having 4 linked CTL epitopes may further comprise a peptide sequence consisting of hydrophilic amino acids, and wherein the peptide having 4 linked CTL epitopes has any one feature selected from the features (1) to (3) below:
   (1) the peptide comprises PEP2 at the C terminus (except for the peptide comprising PEP7 and PEP8 at the N terminus successively disposed in such order from the N terminus via a linker);
   (2) the peptide comprises PEP4 at the C terminus; and
   (3) the peptide comprises PEP10 at the C terminus.
[2] The agent for adjuvant therapy of tumors according to [1], wherein the epitope peptides included in the peptide having 4 linked CTL epitopes are PEP5, PEP6, PEP9, and one epitope peptide selected from the group consisting of PEP2, PEP4, and PEP10, and wherein the C terminus of the peptide having 4 linked CTL epitopes is PEP2, PEP4, or PEP10.
[3] The agent for adjuvant therapy of tumors according to [1] or [2], wherein the peptide having 4 linked CTL epitopes consists of a sequence selected from the sequences indicated below:
   PEP5-(L)-PEP9-(L)-PEP6-(L)-PEP4;
   PEP5-(L)-PEP9-(L)-PEP6-(L)-PEP2; and
   PEP5-(L)-PEP9-(L)-PEP6-(L)-PEP10,
   wherein "-(L)-" represents a linker.
[4] The agent for adjuvant therapy of tumors according to any of [1] to [3], wherein the peptide having 4 linked CTL epitopes consists of the sequence indicated below:
   PEP5-(L)-PEP9-(L)-PEP6-(L)-PEP4,
   wherein "-(L)-" represents a linker.
[5] The agent for adjuvant therapy of tumors according to any of [1] to [4], wherein the linker is an amino acid linker.
[6] The agent for adjuvant therapy of tumors according to [5], wherein the amino acid linker is an arginine dimer or arginine trimer composed of 2 or 3 arginine residues linked to each other.
[7] The agent for adjuvant therapy of tumors according to any of [1] to [6], wherein the peptide sequence consisting of hydrophilic amino acids is linked to the N terminus and is composed of an arginine trimer or arginine tetramer composed of 3 or 4 arginine residues linked to each other.
[8] The agent for adjuvant therapy of tumors according to any of [1] to [7], wherein the peptide having 4 linked CTL epitopes consists of SEQ ID NO: 24, SEQ ID NO: 25, or SEQ ID NO: 26.
[9] The agent for adjuvant therapy of tumors according to any of [1] to [8], wherein the peptide having 4 linked CTL epitopes consists of SEQ ID NO: 24.
[10] An agent for inhibiting tumor metastasis comprising, as an active ingredient, a peptide having 4 linked CTL epitopes comprising 4 CTL epitope peptides linked via linkers, wherein the peptide having 4 linked CTL epitopes comprises the epitope peptide "PEP5" as shown in SEQ ID NO: 5 and 3 epitope peptides selected from the group consisting of the epitope peptide "PEP1" as shown in SEQ ID NO: 1, the epitope peptide "PEP2" as shown in SEQ ID NO: 2, the epitope peptide "PEP4" as shown in SEQ ID NO: 4, the epitope peptide "PEP6" as shown in SEQ ID NO: 6, the epitope peptide "PEP7" as shown in SEQ ID NO: 7, the epitope peptide "PEP8" as shown in SEQ ID NO: 8, the epitope peptide "PEP9" as shown in SEQ ID NO: 9, the epitope peptide "PEP10" as shown in SEQ ID NO: 10, the epitope peptide "PEP13" as shown in SEQ ID NO: 13, the epitope peptide "PEP15" as shown in SEQ ID NO: 15, the epitope peptide "PEP17" as shown in SEQ ID NO: 17, and the epitope peptide "PEP18" as shown in SEQ ID NO: 18 linked via linkers, wherein the peptide having 4 linked CTL epitopes may further comprise a peptide sequence consisting of hydrophilic amino acids, and wherein the peptide having 4 linked CTL epitopes has any one feature selected from the features (1) to (3) below:
   (1) the peptide comprises PEP2 at the C terminus (except for the peptide comprising PEP7 and PEP8 at the N terminus successively disposed in such order from the N terminus via a linker);
   (2) the peptide comprises PEP4 at the C terminus; and
   (3) the peptide comprises PEP10 at the C terminus.
[11] An agent for inhibiting tumor recurrence comprising, as an active ingredient, a peptide having 4 linked CTL epitopes comprising 4 CTL epitope peptides linked via linkers, wherein the peptide having 4 linked CTL epitopes comprises the epitope peptide "PEP5" as shown in SEQ ID NO: 5 and 3 epitope peptides selected from the group consisting of the epitope peptide "PEP1" as shown in SEQ ID NO: 1, the epitope peptide "PEP2" as shown in SEQ ID NO: 2, the epitope peptide "PEP4" as shown in SEQ ID NO: 4, the epitope peptide "PEP6" as shown in SEQ ID NO: 6, the epitope peptide "PEP7" as shown in SEQ ID NO: 7, the epitope peptide "PEP8" as shown in SEQ ID NO: 8, the epitope peptide "PEP9" as shown in SEQ ID NO: 9, the epitope peptide "PEP10" as shown in SEQ ID NO: 10, the epitope peptide "PEP13" as shown in SEQ ID NO: 13, the epitope peptide "PEP15" as shown in SEQ ID NO: 15, the epitope peptide "PEP17" as shown in SEQ ID NO: 17, and the epitope peptide "PEP18" as shown in SEQ ID NO: 18 linked via linkers, wherein the peptide having 4 linked CTL epitopes may further comprise a peptide sequence consisting of hydrophilic amino acids, and wherein the peptide having 4 linked CTL epitopes has any one feature selected from the features (1) to (3) below:
   (1) the peptide comprises PEP2 at the C terminus (except for the peptide comprising PEP7 and PEP8 at the N terminus successively disposed in such order from the N terminus via a linker);
   (2) the peptide comprises PEP4 at the C terminus; and
   (3) the peptide comprises PEP10 at the C terminus.

The present invention also relates to the following embodiments.
- A peptide having 4 linked CTL epitopes defined in [1] to [11], which is used in the adjuvant therapy for tumors.
- A peptide having 4 linked CTL epitopes consisting of SEQ ID NO: 24, which is used in the adjuvant therapy for tumors.
- A peptide having 4 linked CTL epitopes defined in [1] to [11], which is used for inhibition of tumor metastasis.
- A peptide having 4 linked CTL epitopes consisting of SEQ ID NO: 24, which is used for inhibition of tumor metastasis.
- A peptide having 4 linked CTL epitopes defined in [1] to [11], which is used for inhibition of tumor recurrence.
- A peptide having 4 linked CTL epitopes consisting of SEQ ID NO: 24, which is used for inhibition of tumor recurrence.
- A pharmaceutical composition used for the adjuvant therapy for tumors, which comprises the peptide having 4 linked CTL epitopes defined in [1] to [11].
- A pharmaceutical composition used for the adjuvant therapy for tumors, which comprises the peptide having 4 linked CTL epitopes consisting of SEQ ID NO: 24.
- A pharmaceutical composition used for inhibition of tumor metastasis, which comprises the peptide having 4 linked CTL epitopes defined in [1] to [11].
- A pharmaceutical composition used for inhibition of tumor metastasis, which comprises the peptide having 4 linked CTL epitopes consisting of SEQ ID NO: 24.
- A pharmaceutical composition used for inhibition of tumor recurrence, which comprises the peptide having 4 linked CTL epitopes defined in [1] to [11].
- A pharmaceutical composition used for inhibition of tumor recurrence, which comprises the peptide having 4 linked CTL epitopes consisting of SEQ ID NO: 24.
- A method of adjuvant therapy for tumors, which comprises a step of administering the peptide having 4 linked CTL epitopes defined in [1] to [11] in an amount effective for treatment of tumor recurrence and/or metastasis to a patient.
- A method for inhibition of tumor metastasis, which comprises a step of administering the peptide having 4 linked CTL epitopes defined in [1] to [11] in an amount effective for treatment of tumor metastasis to a patient.

A method for inhibition of tumor recurrence, which comprises a step of administering the peptide having 4 linked CTL epitopes defined in [1] to [11] in an amount effective for treatment of tumor recurrence to a patient.

This description includes part or all of the content as disclosed in the description and/or drawings of Japanese Patent Application No. 2019-236947, which is the priority document of the present application.

### Effects of the Invention

The adjuvant therapy for tumors of the present invention enables cancer therapy that exerts inhibitory effects on tumor metastasis and recurrence while suppressing development of side effects. As a consequence, the lifetime of a cancer patient can be prolonged.

### Brief Description of the Drawings

Fig. 1 shows the HPLC chromatogram of the peptide having 4 linked CTL epitopes TPV07.
Fig. 2 shows the HPLC chromatogram of the peptide having 4 linked CTL epitopes TPV08.
Fig. 3 shows the mass spectrum of the peptide having 4 linked CTL epitopes TPV07.
Fig. 4 shows the mass spectrum of the peptide having 4 linked CTL epitopes TPV08.
Fig. 5 shows effects of TPV06 for inhibiting the number of metastatic pulmonary nodules in mouse models subjected to intravenous transplantation of the B16F10.A24/SART2⁹³⁻¹⁰¹ cells.

### Embodiments of the Invention

The present invention relates to an agent for adjuvant therapy of tumors comprising, as an active ingredient, the peptide having 4 linked CTL epitopes. The present invention also relates to an agent for inhibiting tumor metastasis comprising, as an active ingredient, the peptide having 4 linked CTL epitopes. The present invention also relates to an agent for inhibiting tumor recurrence comprising, as an active ingredient, the peptide having 4 linked CTL epitopes.

The term "the peptide having 4 linked CTL epitopes" used in the present invention refers to a peptide comprising 4 peptides selected from among CTL epitope peptides derived from the same and/or different tumor antigen molecules that are linearly linked to each other via linkers to form one molecule.

Examples of known CTL epitope peptides derived from tumor antigen molecules include the following:
KLVERLGAA (SEQ ID NO: 1; referred to as "PEP1" herein, e.g., WO 2001/011044);
ASLDSDPWV (SEQ ID NO: 2; referred to as "PEP2" herein, e.g., WO 2002/010369);
ALVEFEDVL (SEQ ID NO: 3; referred to as "PEP3" herein, e.g., WO 2002/010369);
LLQAEAPRL (SEQ ID NO: 4; referred to as "PEP4" herein, e.g., WO 2000/12701);
DYSARWNEI (SEQ ID NO: 5; referred to as "PEP5" herein, e.g., JP H11-318455 A);
VYDYNCHVDL (SEQ ID NO: 6; referred to as "PEP6" herein, e.g., WO 2000/12701);
LYAWEPSFL (SEQ ID NO: 7; referred to as "PEP7 " herein, e.g., JP 2003-000270 A);
DYLRSVLEDF (SEQ ID NO: 8; referred to as "PEP8" herein, e.g., WO 2001/011044);
QIRPIFSNR (SEQ ID NO: 9; referred to as "PEP9" herein, e.g., WO 2008/007711);
ILEQSGEWWK (SEQ ID NO: 10; referred to as "PEP10" herein, e.g., WO 2009/022652);
VIQNLERGYR (SEQ ID NO: 11; referred to as "PEP11" herein, e.g., WO 2009/022652);
KLKHYGPGWV (SEQ ID NO: 12; referred to as "PEP12" herein, e.g., WO 1999/067288);
RLQEWCSVI (SEQ ID NO: 13; referred to as "PEP13" herein, e.g., WO 2002/010369);
ILGELREKV (SEQ ID NO: 14; referred to as "PEP14" herein, e.g., WO 2002/010369);
DYVREHKDNI (SEQ ID NO: 15; referred to as "PEP15" herein, e.g., WO 2005/071075);
HYTNASDGL (SEQ ID NO: 16; referred to as "PEP16" herein, e.g., WO 2001/011044);
NYSVRYRPGL (SEQ ID NO: 17; referred to as "PEP17" herein, e.g., JP 2003-000270 A); and
RYLTQETNKV (SEQ ID NO: 18; referred to as "PEP18" herein, e.g., WO 2005/116056).

Table 1 shows information concerning proteins from which the CTL epitope peptides PEP1 to PEP18 are derived. These proteins have been reported to be expressed at high levels in tumor tissue.

**[Table 1]**

| Peptide | Origin | Amino acid sequence | SEQ ID NO: |
|---|---|---|---|
| PEP1 | Lck-246 | KLVERLGAA | SEQ ID NO: 1 |
| PEP2 | WHSC2-103 | ASLDSDPWV | SEQ ID NO: 2 |
| PEP3 | HNRPL-140 | ALVEFEDVL | SEQ ID NO: 3 |
| PEP4 | SART3-302 | LLQAEAPRL | SEQ ID NO: 4 |
| PEP5 | SART2-93 | DYSARWNEI | SEQ ID NO: 5 |
| PEP6 | SART3-109 | VYDYNCHVDL | SEQ ID NO: 6 |
| PEP7 | MRP3-503 | LYAWEPSFL | SEQ ID NO: 7 |
| PEP8 | Lck-488 | DYLRSVLEDF | SEQ ID NO: 8 |
| PEP9 | SART3-734 | QIRPIFSNR | SEQ ID NO: 9 |
| PEP10 | Lck-90 | ILEQSGEWWK | SEQ ID NO: 10 |
| PEP11 | Lck-449 | VIQNLERGYR | SEQ ID NO: 11 |
| PEP12 | CypB-129 | KLKHYGPGWV | SEQ ID NO: 12 |
| PEP13 | UBE2V-43 | RLQEWCSVI | SEQ ID NO: 13 |
| PEP14 | WHSC2-141 | ILGELREKV | SEQ ID NO: 14 |
| PEP15 | EGFR-800 | DYVREHKDNI | SEQ ID NO: 15 |
| PEP16 | Lck-208 | HYTNASDGL | SEQ ID NO: 16 |
| PEP17 | MRP3-1293 | NYSVRYRPGL | SEQ ID NO: 17 |
| PEP18 | PTHrP-102 | RYLTQETNKV | SEQ ID NO: 18 |

The peptide having 4 linked CTL epitopes of the present invention comprises 4 types of CTL epitope peptides selected from among particular 13 types of the CTL epitope peptides: the peptide "PEP1" as shown in SEQ ID NO: 1, the peptide "PEP2" as shown in SEQ ID NO: 2, the peptide "PEP4" as shown in SEQ ID NO: 4, the peptide "PEP5" as shown in SEQ ID NO: 5, the peptide "PEP6" as shown in SEQ ID NO: 6, the peptide "PEP7" as shown in SEQ ID NO: 7, the peptide "PEP8" as shown in SEQ ID NO: 8, the peptide "PEP9" as shown in SEQ ID NO: 9, the peptide "PEP10" as shown in SEQ ID NO: 10, the peptide "PEP13" as shown in SEQ ID NO: 13, the peptide "PEP15" as shown in SEQ ID NO: 15, the peptide "PEP17" as shown in SEQ ID NO: 17, and the peptide "PEP18" as shown in SEQ ID NO: 18, linearly linked via linkers. The peptide having 4 linked CTL epitopes can induce and/or activate 3 or more CTLs specific for relevant CTL epitope peptides. Peptides may not be directly evaluated concerning CTL epitope-peptide-specific induction. The occurrence of epitope-peptide-specific CTL induction can be determined by cleavage experiment with immunoproteasomes (e.g., WO 2015/060235).

In the present invention, the peptide having 4 linked CTL epitopes comprises:
the peptide "PEP5" as shown in SEQ ID NO: 5, and
3 peptides selected from the group consisting of the peptide "PEP1" as shown in SEQ ID NO: 1, the peptide "PEP2" as shown in SEQ ID NO: 2, the peptide "PEP4" as shown in SEQ ID NO: 4, the peptide "PEP6" as shown in SEQ ID NO: 6, the peptide "PEP7" as shown in SEQ ID NO: 7, the peptide "PEP8" as shown in SEQ ID NO: 8, the peptide "PEP9" as shown in SEQ ID NO: 9, the peptide "PEP10" as shown in SEQ ID NO: 10, the peptide "PEP13" as shown in SEQ ID NO: 13, the peptide "PEP15" as shown in SEQ ID NO: 15, the peptide "PEP17" as shown in SEQ ID NO: 17, and the peptide "PEP18" as shown in SEQ ID NO: 18 linked via linkers.

In the present invention, a peptide having an amino acid sequence having substitution, insertion, deletion, and/or addition of one or a plurality of amino acids in the amino acid sequence of PEP1, PEP2, PEP4, PEP5, PEP6, PEP7, PEP8, PEP9, PEP10, PEP13, PEP15, PEP17, or PEP18 and having the capacity for inducing CTL and/or the capacity for inducing immunoglobulin production equivalent to or higher than those of the original peptide can be used as a "CTL epitope peptide." The term "plurality" used herein refers to 2 or 3, and preferably 2. An example of such peptide is a peptide obtained by substitution with amino acids having properties similar to those of the original amino acids (i.e., a peptide obtained by conservative amino acid substitution).

Whether or not a peptide of interest is a "peptide having the capacity for inducing CTL and/or the capacity for inducting immunoglobulin production equivalent to or higher than those of the original peptide" can be determined in accordance with, for example, the method disclosed in WO 2015/060235. According to the method disclosed in WO 2015/060235, the capacity for inducing CTL is evaluated using, as the indicator, the number of IFN-γ-producing cells in wells supplemented with cells obtained from a mouse to which a test peptide having an amino acid sequence derived by substitution, insertion, deletion, and/or addition of one or a plurality of amino acids from the original sequence has been administered in advance, antigen-presenting cells derived from a mouse of the same lineage, and the test peptide. When the result of evaluation is equivalent to or higher than the Δ value of the original peptide (positive: 10 ≤ Δ < 100; moderately positive: 100 ≤ Δ < 200; strongly positive: 200 ≤ Δ), the peptide of interest can be determined to have the capacity for inducing CTL equivalent to or higher than that of the original peptide. When the original peptide is evaluated "positive" and a peptide having an amino acid sequence derived by substitution, insertion, deletion, and/or addition of one or a plurality of amino acids from the original sequence is also evaluated "positive," the capacity for inducing CTL is considered equivalent. The capacity for inducting immunoglobulin production is evaluated using, as the indicator, the CTL-epitope-specific IgG antibody titer in the serum of the mouse to which the test peptide has been administered. When an increase in the obtained IgG antibody titer (fold) is equivalent to or higher than that of the original peptide (2 < fold < 10, 10 ≤ fold < 100, 100 ≤ fold), the peptide of interest can be determined to have the capacity for inducting immunoglobulin production equivalent to or higher than that of the original peptide. When the result of the measurement on the original peptide is within the range of "2 < fold < 10" and the result of the measurement on the peptide having an amino acid sequence derived by substitution, insertion, deletion, and/or addition of one or a plurality of amino acids from the original sequence is also within the range of "2 < fold < 10," the capacity for inducting immunoglobulin production is considered equivalent.

In the present invention, any linker can be used, provided that it is cleaved upon administration of a peptide having 4 linked CTL epitopes to an organism and that the linked CTL epitope peptides can be separated from each other. Examples thereof include an ester bond, an ether bond, an amide bond, a sugar chain linker, a polyethylene glycol linker, and an amino acid linker. Examples of amino acid sequences used as amino acid linkers include an arginine dimer (RR), an arginine trimer (RRR), an arginine tetramer (RRRR), a lysine dimer (KK), a lysine trimer (KKK), a lysine tetramer (KKKK), a glycine dimer (GG), a glycine trimer (GGG), a glycine tetramer (GGGG), a glycine pentamer (GGGGG), a glycine hexamer (GGGGGG), alanine-alanine-tyrosine (AAY), isoleucine-leucine-alanine (ILA), and arginine-valine-lysine-arginine (RVKR), with an arginine dimer (RR) or trimer (RRR) being preferable and an arginine dimer (RR) being more preferable. Linkers used for an epitope-linked peptide are known in the art, and a person skilled in the art can use an adequately selected linker.

In the peptide having 4 linked CTL epitopes of the present invention, CTL epitope peptides to be selected and the arrangements thereof can be determined by administering a peptide having 4 linked CTL epitopes synthesized in given combinations and in a given order of epitopes to human HLA-A-expressing transgenic mice and evaluating the occurrence of CTL-epitope-peptide-specific CTL induction *in vivo.* The occurrence of CTL-epitope-peptide-specific CTL induction *in vivo* can be evaluated in accordance with, for example, the method disclosed in WO 2015/060235. In accordance with such method, at least 3, and preferably 4 types of CTL epitope peptides for which CTL induction specific for relevant CTL epitope peptides was observed can be selected, and the arrangements thereof can be determined.

The peptide having 4 linked CTL epitopes of the present invention preferably has a feature selected from among (1) to (3) below:
(1) the peptide comprises PEP2 at the C terminus (except for the peptide comprising PEP7 and PEP8 at the N terminus successively disposed in such order from the N terminus via a linker);
(2) the peptide comprises PEP4 at the C terminus; and
(3) the peptide comprises PEP10 at the C terminus.

Accordingly, the peptide having 4 linked CTL epitopes of the present invention preferably comprises the peptide "PEP5" as shown in SEQ ID NO: 5 and 3 peptides selected from the group consisting of the peptide "PEP1" as shown in SEQ ID NO: 1, the peptide "PEP2" as shown in SEQ ID NO: 2, the peptide "PEP4" as shown in SEQ ID NO: 4, the peptide "PEP6" as shown in SEQ ID NO: 6, the peptide "PEP7" as shown in SEQ ID NO: 7, the peptide "PEP8" as shown in SEQ ID NO: 8, the peptide "PEP9" as shown in SEQ ID NO: 9, the peptide "PEP10" as shown in SEQ ID NO: 10, the peptide "PEP13" as shown in SEQ ID NO: 13, the peptide "PEP15" as shown in SEQ ID NO: 15, the peptide "PEP17" as shown in SEQ ID NO: 17, and the peptide "PEP18" as shown in SEQ ID NO: 18 linked via linkers, and has a feature selected from among (1) to (3) below:
(1) the peptide comprises PEP2 at the C terminus (except for the peptide comprising PEP7 and PEP8 at the N terminus successively disposed in such order from the N terminus via a linker);
(2) the peptide comprises PEP4 at the C terminus; and
(3) the peptide comprises PEP10 at the C terminus.

More preferably, the peptide having 4 linked CTL epitopes of the present invention comprises the peptide "PEP5" as shown in SEQ ID NO: 5, the peptide "PEP6" as shown in SEQ ID NO: 6, the peptide "PEP9" as shown in SEQ ID NO: 9, and one peptide selected from the group consisting of the peptide "PEP2" as shown in SEQ ID NO: 2, the peptide "PEP4" as shown in SEQ ID NO: 4, and the peptide "PEP10" as shown in SEQ ID NO: 10 linked via linkers, and comprises PEP2, PEP4, or PEP10 at the C terminus.

More preferably, the peptide having 4 linked CTL epitopes of the present invention consists of a sequence selected from the followings, wherein "-(L)-" represents a linker:
PEP5-(L)-PEP6-(L)-PEP9-(L)-PEP4;
PEP9-(L)-PEP5-(L)-PEP6-(L)-PEP4;
PEP6-(L)-PEP5-(L)-PEP9-(L)-PEP4;
PEP6-(L)-PEP9-(L)-PEP5-(L)-PEP4;
PEP9-(L)-PEP6-(L)-PEP5-(L)-PEP4;
PEP5-(L)-PEP9-(L)-PEP6-(L)-PEP4;
PEP5-(L)-PEP9-(L)-PEP6-(L)-PEP2; and
PEP5-(L)-PEP9-(L)-PEP6-(L)-PEP10.

More preferably, the peptide having 4 linked CTL epitopes of the present invention consists of a sequence selected from the followings, wherein "-(L)-" represents a linker:
PEP5-(L)-PEP9-(L)-PEP6-(L)-PEP4;
PEP5-(L)-PEP9-(L)-PEP6-(L)-PEP2; and
PEP5-(L)-PEP9-(L)-PEP6-(L)-PEP10.

More preferably, the peptide having 4 linked CTL epitopes of the present invention consists of a sequence selected from the followings, wherein the linker is an arginine dimer:
PEP5-RR-PEP9-RR-PEP6-RR-PEP4 (SEQ ID NO: 24, referred to as "TPV06" herein);
PEP5-RR-PEP9-RR-PEP6-RR-PEP2 (SEQ ID NO: 25, referred to as "TPV07" herein); and
PEP5-RR-PEP9-RR-PEP6-RR-PEP10 (SEQ ID NO: 26, referred to as "TPV08" herein).
Further preferably, the peptide having 4 linked CTL epitopes of the present invention is the peptide TPV06 as shown in SEQ ID NO: 24.

In an aspect of the present invention, the peptide having 4 linked CTL epitopes is preferably a peptide having a sequence selected from among SEQ ID NOs: 19 to 28 shown in Table 2 below.

**[Table 2]**

| Peptide | Amino acid sequence | SEQ ID NO: |
|---|---|---|
| TPV01 | PEP5-RR-PEP6-RR-PEP9-RR-PEP4 | SEQ ID NO: 19 |
| TPV02 | PEP9-RR-PEP5-RR-PEP6-RR-PEP4 | SEQ ID NO: 20 |
| TPV03 | PEP6-RR-PEP5-RR-PEP9-RR-PEP4 | SEQ ID NO: 21 |
| TPV04 | PEP6-RR-PEP9-RR-PEP5-RR-PEP4 | SEQ ID NO: 22 |
| TPV05 | PEP9-RR-PEP6-RR-PEP5-RR-PEP4 | SEQ ID NO: 23 |
| TPV06 | PEP5-RR-PEP9-RR-PEP6-RR-PEP4 | SEQ ID NO: 24 |
| TPV07 | PEP5-RR-PEP9-RR-PEP6-RR-PEP2 | SEQ ID NO: 25 |
| TPV08 | PEP5-RR-PEP9-RR-PEP6-RR-PEP10 | SEQ ID NO: 26 |
| TPV09 | RRR-PEP5-RR-PEP9-RR-PEP6-RR-PEP4 | SEQ ID NO: 27 |
| TPV10 | RRRR-PEP5-RR-PEP9-RR-PEP6-RR-PEP4 | SEQ ID NO: 28 |

More preferably, the peptide having 4 linked CTL epitopes is the peptide indicated below, which is selected from among those shown in Table 2:
- TPV01 (SEQ ID NO: 19);
- TPV02 (SEQ ID NO: 20);
- TPV03 (SEQ ID NO: 21);
- TPV04 (SEQ ID NO: 22);
- TPV05 (SEQ ID NO: 23); or
- TPV06 (SEQ ID NO: 24).

In the present invention, 2 or more types of peptides having 4 linked CTL epitopes may be used. For example, 2, 3, 4, or more types of peptides having 4 linked CTL epitopes may be used separately or in a mixed form. It is preferable that 3 types of peptides having 4 linked CTL epitopes be used in a mixed form. As long as at least one peptide having 4 linked CTL epitopes of the present invention is included in 2 or more types of peptides having 4 linked CTL epitopes to be used, other peptides may be the peptides having 4 linked CTL epitopes of the present invention or the peptides having 4 linked CTL epitopes as described in, for example, WO 2015/060235. It is preferable that the peptide as shown in SEQ ID NO: 24 be used in combination with one or more of the peptides having 4 linked CTL epitopes as described in WO 2015/060235, it is more preferable that the peptide as shown in SEQ ID NO: 24 be used in combination with 2 types of the peptides having 4 linked CTL epitopes as described in WO 2015/060235, and it is further preferable that the peptide as shown in SEQ ID NO: 24, the peptide as shown in SEQ ID NO: 29 (TPV011: PEP15-RR-PEP18-RR-PEP1-RR-PEP10), and the peptide as shown in SEQ ID NO: 30 (TPV012: RRRR-PEP7-RR-PEP13-RR-PEP8-RR-PEP2) be used in a mixed form.

The peptide having 4 linked CTL epitopes of the present invention may further have a peptide sequence consisting of hydrophilic amino acids. Such peptide sequence can be added to the N terminus and/or the C terminus of the peptide having 4 linked CTL epitopes, and it is preferably added to the N terminus. Such peptide sequence can consist of 1 to 15, preferably 2 to 10, and more preferably 3 to 5 hydrophilic amino acids selected from the group consisting of arginine, histidine, lysine, threonine, tyrosine, serine, asparagine, glutamine, aspartic acid, and glutamic acid. Examples of peptide sequences that can be used include an arginine trimer (RRR) and an arginine tetramer (RRRR). Examples of peptides having 4 linked CTL epitopes comprising the peptide sequences as described above added thereto include RRR-TPV06, RRRR-TPV06, TPV06-RRR, TPV06-RRRR, RRR-TPV07, RRRR-TPV07, TPV07-RRR, TPV07-RRRR, RRR-TPV08, RRRR-TPV08, TPV08-RRR, TPV08-RRRR, KKK-TPV06, KKKK-TPV06, TPV06-KKK, TPV06-KKKK, KKK-TPV07, KKKK-TPV07, TPV07-KKK, TPV07-KKKK, KKK-TPV08, KKKK-TPV08, TPV08-KKK, TPV08-KKKK, HHH-TPV06, HHHH-TPV06, TPV06-HHH, TPV06-HHHH, HHH-TPV07, HHHH-TPV07, TPV07-HHH, TPV07-HHHH, HHH-TPV08, HHHH-TPV08, TPV08-HHH, TPV08-HHHH, RRKK-TPV06, RKRK-TPV06, RHRH-TPV06, RRHH-TPV06, KKHH-TPV06, and KHKH-TPV06. Preferable examples include RRR-TPV06, RRRR-TPV06, RRR-TPV07, RRRR-TPV07, RRR-TPV08, RRRR-TPV08, KKK-TPV06, KKKK-TPV06, KKK-TPV07, KKKK-TPV07, KKK-TPV08, KKKK-TPV08, HHH-TPV06, HHHH-TPV06, HHH-TPV07, HHHH-TPV07, HHH-TPV08, HHHH-TPV08, RRKK-TPV06, RKRK-TPV06, RHRH-TPV06, RRHH-TPV06, KKHH-TPV06, and KHKH-TPV06. More preferable examples include RRR-TPV06 (TPV09), RRRR-TPV06 (TPV10), RRR-TPV07, RRRR-TPV07, RRR-TPV08, and RRRR-TPV08. The most preferable examples include RRR-TPV06 (TPV09) and RRRR-TPV06 (TPV10).

A peptide comprising a peptide sequence consisting of hydrophilic amino acids is known to have improved solubility in an aqueous solvent (Peptides 38, 302-311, 2012; JP 2006-188507 A). With the addition of such peptide sequence to the peptide having 4 linked CTL epitopes of the present invention, a degree of solubility of the peptide having 4 linked CTL epitopes in an aqueous solvent can be improved.

"The peptide having 4 linked CTL epitopes that may further comprise a peptide sequence consisting of hydrophilic amino acids" of the present invention is preferably the peptide having 4 linked CTL epitopes that may comprise a peptide sequence consisting of hydrophilic amino acids at the N terminus, more preferably the peptide having 4 linked CTL epitopes that may comprise a peptide sequence consisting of 3 to 5 hydrophilic amino acids selected from the group consisting of arginine, histidine, and lysine at the N terminus, further preferably the peptide having 4 linked CTL epitopes that may comprise an arginine trimer (RRR) or arginine tetramer (RRRR) at the N terminus, and still further preferably the peptide having 4 linked CTL epitopes that does not comprise a peptide sequence consisting of hydrophilic amino acids.

The peptide having 4 linked CTL epitopes of the present invention can be synthesized in accordance with, for example, the method disclosed in WO 2015/060235.

In the present invention, "adjuvant therapy" is a method for treatment or inhibition of tumor recurrence and/or metastasis after surgery. The adjuvant therapy of the present invention comprises a step of administering the peptide having 4 linked CTL epitopes of the present invention to a patient after tumor resection.

While a target patient is not limited in the present invention, advantageous effects can be achieved in humans, in particular.

The recommended dose of the peptide having 4 linked CTL epitopes of the present invention in human is preferably in a range of 3 to 9 mg/body/day per one peptide having 4 linked CTL epitopes.

The term "recommended dose" used herein refers to a dose which can be used safely without developing any serious side effects and can exert maximal therapeutic effects, determined on the basis of clinical testing and the like. Specifically, the "recommended dose" is that approved, recommended, and advised by public organizations or institutions, such as the Pharmaceuticals and Medical Devices Agency (PMDA), Japan, the Food and Drug Administration (FDA), U.S.A., or the European Medicines Agency (EMA), and described in, for example, the product information, the interview form, or treatment guidelines. The "recommended dose" is preferably a dose approved by any of PMDA, FDA, or EMA.

The administration schedule of the agent for adjuvant therapy of tumors, the agent for inhibiting tumor metastasis, and the agent for inhibiting tumor recurrence of the present invention can be adequately determined in accordance with a tumor type, a disease stage, and other conditions.

The administration of the peptide having 4 linked CTL epitopes is preferably scheduled to comprise a cycle of 21 days in total in which a step of single administration per week is repeated 3 times (once a day on Day 1, Day 8, and Day 15). After the third cycle, administration is preferably scheduled to comprise a cycle of 21 days in total in which drug administration on Day 1 is followed by drug holidays for 20 days (single administration in 3 weeks).

The frequency of administration of the peptide having 4 linked CTL epitopes of the present invention per day can be adequately determined in accordance with a cancer type, a disease stage, and other conditions. The peptide having 4 linked CTL epitopes is preferably administered once a day.

Target tumors to be treated in the present invention are not particularly limited, provided that anti-tumor effects can be exerted in the adjuvant therapy for tumors. Target tumors are preferably tumors on which the peptides having 4 linked CTL epitopes exert anti-tumor effects in the adjuvant therapy for tumors, and more preferably Lck-, WHSC2-, SART2-, SART3-, MRP3-, UBE2V-, EGFR-, or PTHrP-positive malignant tumors, and more preferably SART2-positive malignant tumors.

Specific examples of target tumors of the present invention include brain tumor, head and neck cancer, digestive system cancer (e.g., esophageal cancer, gastric cancer, duodenal cancer, liver cancer, bile duct cancer (e.g., gallbladder cancer and bile duct cancer), pancreatic cancer, small intestinal cancer, large bowel cancer (e.g., colorectal cancer, colon cancer, and rectal cancer), and gastrointestinal stromal tumor), lung cancer (e.g., non-small cell lung cancer and small cell lung cancer), breast cancer, ovarian cancer, uterine cancer (e.g., cervical cancer and uterine body cancer), renal cancer, urothelial cancer (e.g., bladder cancer, renal pelvic cancer, and ureteral cancer), prostate cancer, skin cancer, and cancer of unknown primary. Cancer encompasses primary cancer and cancer metastasized to other organs (e.g., liver). From the viewpoint of anti-tumor effects, preferable targets are head and neck cancer, digestive system cancer, lung cancer, renal cancer, urothelial cancer, and skin cancer, more preferable targets are digestive system cancer, lung cancer, urothelial cancer, and skin cancer, and particularly preferable targets are lung cancer and urothelial cancer.

Dosage forms of the agent for adjuvant therapy of tumors, the agent for inhibiting tumor metastasis, and the agent for inhibiting tumor recurrence of the present invention are not particularly limited and can be adequately selected in accordance with the purpose of treatment. Specific examples include oral preparations (e.g., tablets, coated tablets, powders, granules, capsules, and liquid preparations), injections, suppositories, patches, and ointments, with injections being preferable.

The agent for adjuvant therapy of tumors, the agent for inhibiting tumor metastasis, and the agent for inhibiting tumor recurrence of the present invention can be prepared with the use of a pharmaceutically acceptable carrier in accordance with a conventional technique. Examples of pharmaceutical carriers include various types of carriers that are commonly used for medicines, such as an excipient, a binder, a disintegrator, a lubricant, a diluent, a solubilizer, a suspending agent, an isotonizing agent, a pH modifier, a buffer, a stabilizer, a colorant, a corrigent, and a flavoring agent.

The peptide having 4 linked CTL epitopes as an active ingredient of the agent for adjuvant therapy, the agent for inhibiting tumor metastasis, and the agent for inhibiting tumor recurrence of the present invention can selectively and specifically target cells expressing the epitope included therein. In comparison with anti-cancer agents used as an agent for adjuvant therapy, an agent for inhibiting tumor metastasis, and an agent for inhibiting tumor recurrence of conventional techniques, accordingly, the agent for adjuvant therapy, the agent for inhibiting tumor metastasis, and the agent for inhibiting tumor recurrence of the present invention can be used effectively without developing serious side effects while alleviating stress imposed on a patient.

Anti-cancer effects are often verified with the use of animal models. In general, the growth of transplanted tumors or the survival rate of animals to which tumors have been transplanted is used as the indicator. In contrast, the effects of the agent for adjuvant therapy, the agent for inhibiting tumor metastasis, and the agent for inhibiting tumor recurrence may be more preferably verified by examining the effects exerted on metastasis rather than by resecting the tumors that have been once transplanted into animal models. Accordingly, the effects of the agent for adjuvant therapy of tumors, the agent for inhibiting tumor metastasis, and the agent for inhibiting tumor recurrence of the present invention can be verified with the use of, for example, animal models that develop tumors at sites different from the site of tumor transplantation; i.e., animal models that develop metastasis.

In addition, the peptide having 4 linked CTL epitopes as an active ingredient of the agent for adjuvant therapy, the agent for inhibiting tumor metastasis, and the agent for inhibiting tumor recurrence of the present invention has the capacity for induction of HLA-A-restricted CTL responses. Accordingly, animal models used to verify the effects are required to express the HLA-A molecules.

An example of an animal model is, but is not particularly limited to, a non-immunodeficient non-human animal having the knocked-in HLA-A gene. A non-immunodeficient non-human animal having the knocked-in HLA-A gene is not limited, as long as it has human HLA-A α1 and α2 regions. An example thereof that can be preferably used is the human HLA-A24 gene knocked-in mouse (B2m^{tm2(HLA-A24/H-2Db/B2M)Tai} mouse) disclosed in WO 2015/056774 and J. Immunol.: 198, 516-527, 2017.

### Examples

Hereafter, the present invention is described in greater detail with reference to the examples, although the present invention is not limited to these examples. A person skilled in the art would be able to make various forms of modification within the technical scope of the present invention.

### Example 1: Peptide synthesis and purification

Peptides having 4 linked epitopes TPV07 and TPV08 were synthesized using an automatic peptide synthesizer Prelude (Protein Technologies, Inc.) by the technique of 9-fluorenylmethyl-oxycarbonyl (Fmoc) solid-phase peptide synthesis. Specifically, an amino acid comprising an α-amino group protected with an Fmoc group and a side-chain functional group protected with a common protective group was fused to the Wang-ChemMatrix resin under the 1-(mesitylene-2-sulfonyl)-3-nitro-1,2,4-triazole/N-methylimidazole/dichloromethane conditions to support the C-terminal amino acid on the resin. A deblocking solution (20% piperidine/N,N-dimethylformamide (DMF)) was injected thereinto so as to remove the Fmoc group, and an amino acid comprising an α-amino group protected with an Fmoc group and a side-chain functional group protected with a common protective group was fused thereto under the 1-[bis(dimethylamino)methylene]-5-chloro-1H-benzo-triazolium 3-oxide hexafluorophosphate (HCTU)/N-methylmorpholine (NMM)/DMF conditions to synthesize a dipeptide. Fmoc deprotection with the deblocking solution and amino acid fusion under HCTU/NMM/DMF conditions were repeated to synthesize peptides each comprising a sequence of interest. After the completion of synthesis of the protected peptide resin, a deprotecting solution (2.5% triisopropylsilane, 2.5% water, 2.5% 1,2-ethanedithiol, 92.5% trifluoroacetic acid) was added to the protected peptide resin, and the reaction was allowed to proceed for 4 hours to remove the peptide side chain protective group and to cleave a free peptide from the resin. The resin was removed by filtration, the filtrate was added to cold ether, and the peptides were recovered as precipitates. The synthetic peptides were purified through the Proteonavi column (Shiseido) using a solvent system of an aqueous solution of 0.1% TFA and acetonitrile. The purity of the final form of the purified peptides was examined using the CAPCELL PAK UG120 column (Shiseido) and the HPLC system (Hitachi). After the molecular weight was examined by the ESI-MS system (Synapt HDMS, WATERS), the peptides were lyophilized, stored at cool temperature in the dark, and then subjected to the examples descried below.

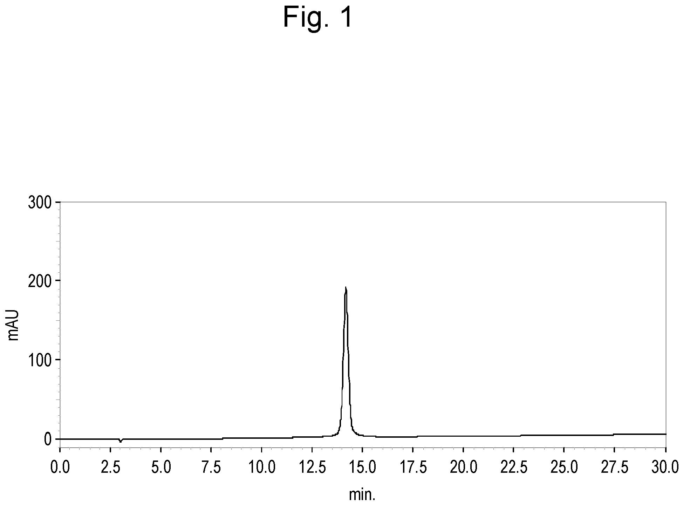

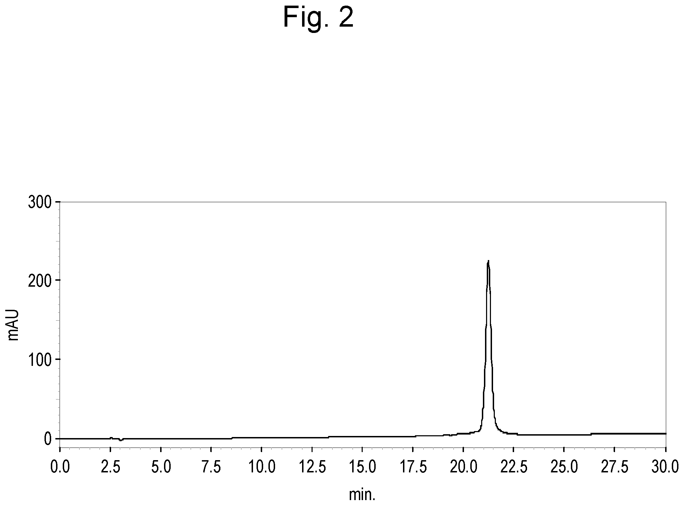

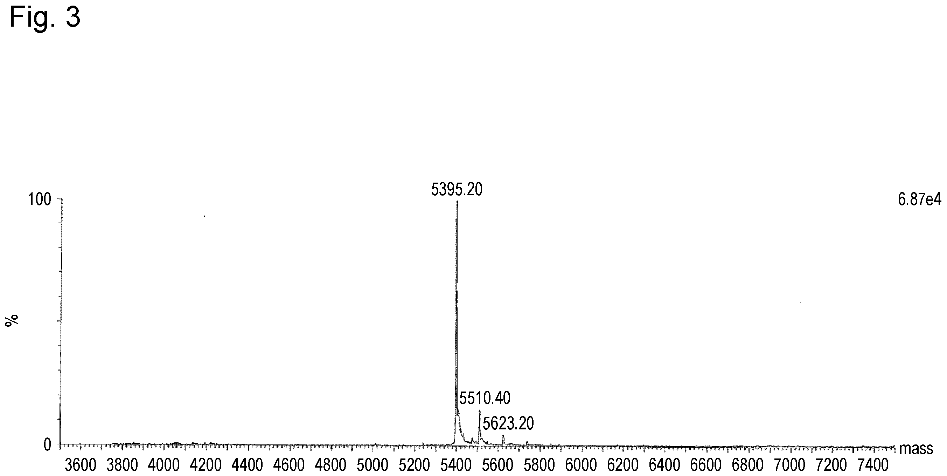

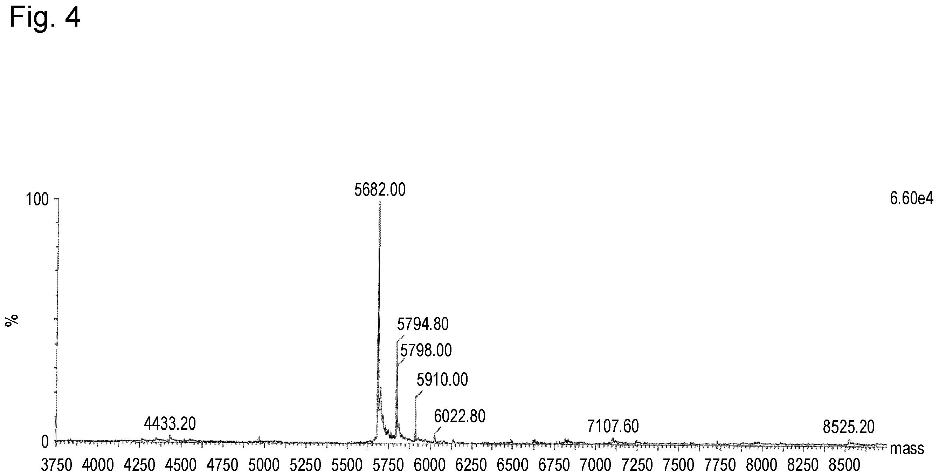

Table 3 shows the molecular weights of TPV07 and TPV08 measured by mass spectral (MS) analysis. Figs. 1 to 4 show the HPLC chromatograms and MS data of TPV07 and TPV08.

**[Table 3]**

| Peptide | Molecular weight (Calculated) | Molecular weight (Deconvoluted) |
|---|---|---|
| TPV07 | 5396.03 | 5395.20 |
| TPV08 | 5682.41 | 5682.00 |

TPV01 to TPV06 and TPV09 to TPV12 can be synthesized in accordance with, for example, the method described in WO 2015/060235 or the method employed for TPV07 and TPV08. All the peptides had the purity of 90% or higher.

### Example 2: Effects of TPV06 on lung metastatic models of B16F10.A24/SART2⁹³⁻¹⁰¹ cells

Eleven-week-old B2m^{tm2(HLA-A24/H-2Db/B2M)Tai} mice (J. Immunol.: 198, 516-527, 2017) were randomly divided into groups each consisting of 10 mice. The day of grouping was designated Day 0.

The B16F10.A24/SART2⁹³⁻¹⁰¹ cells (PLoS One: 13, e0199249, 2018) were cultured using the Dulbecco's Modified Eagle's Medium (Sigma-Aldrich) containing 10% FBS. The B16F10.A24/SART2⁹³⁻¹⁰¹ cells were subjected to subculture in an incubator at 37°C in the presence of 5% CO₂ two times a week at a ratio of 1:25 to 1:40.

On the day of grouping of mice (Day 0), the B16F10.A24/SART2⁹³⁻¹⁰¹ cell suspension prepared using D-PBS (FUJIFILM Wako Pure Chemical Corporation) was transplanted into the tail veins of mice at 1 × 10⁶ cells/0.1 ml.

The mouse models are found to undergo recurrence of tumor cells transplanted into the blood in the lung. Accordingly, such mouse models can be regarded as simulating the recurrence mechanism caused by cancer metastasis in humans. By verifying the effects in such models, accordingly, the inhibitory effects on tumor metastasis and recurrence can be verified.

The peptide having 4 linked epitopes TPV06 (SEQ ID NO: 24) was dissolved in distilled water (Otsuka Pharmaceutical Factory Inc.) to prepare a solution of 6 mg/ml peptides, and the resulting solution was filled into the B Braun Injekt syringe (B. Braun Aesculap). After the equivalent amount of Montanide ISA 51 VG (SEPPIC) was filled into another syringe, these syringes were connected to each other using a connector, and the peptide solution was thoroughly mixed with Montanide ISA 51 VG to prepare an emulsion. As a control sample, distilled water was mixed with the equivalent amount of Montanide ISA 51 VG to prepare an emulsion. The emulsions were weekly administered subcutaneously in amounts of 100 µl each in the vicinity of the bases of the tails of the B2m^{tm2(HLA-A24/H-2Db/B2M)Tai} mice, and administration was performed 3 times in total (on Day 1, Day 8, and Day 15).

The lungs were resected 28 days after transplantation of the B16F10.A24/SART2⁹³⁻¹⁰¹ cells (Day 28), and the number of metastatic pulmonary nodules was visually counted.

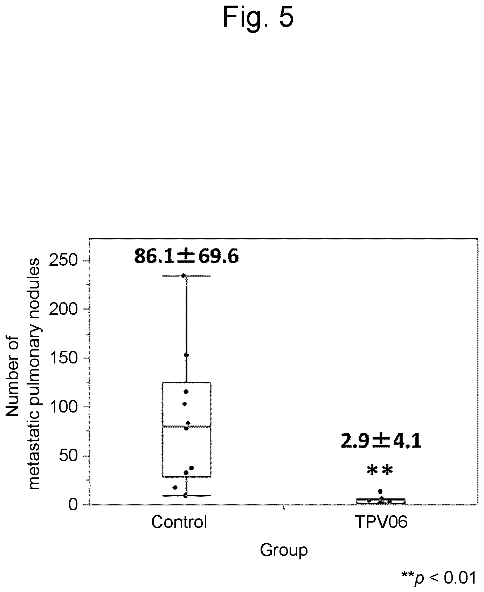

As shown in Fig. 5, the number of metastatic pulmonary nodules was significantly reduced in the group of TPV06 administration, compared with the control group (p = 0.0014, Student's t-test).

The results demonstrate that administration of TPV06 can inhibit metastasis and recurrence of SART2⁹³⁻¹⁰¹-expressing tumor cells to a significant extent. Thus, the peptide having 4 linked CTL epitopes is useful for treatment of tumor metastasis and recurrence.

In general, antigen-specific CTLs are known to specifically recognize, activate, and kill the cells presenting cancer-antigen-derived CTL epitope peptides via HLA molecules on the cell surface (Janeway's Immunobiology, 7th edition). Accordingly, if the target cells express cancer antigens to be targeted by the peptide having 4 linked CTL epitopes, the peptide having 4 linked CTL epitopes can exert anti-tumor effects. The peptide having 4 linked CTL epitopes can exert the inhibitory effects on tumor metastasis and recurrence (therapeutic effects) if the cells express the cancer antigens targeted by the peptide having 4 linked CTL epitopes. Thus, types of target tumors to be treated are not limited.

All publications, patents, and patent applications cited herein are incorporated herein

## Claims

1. An agent for adjuvant therapy of tumors comprising, as an active ingredient, a peptide having 4 linked CTL epitopes comprising 4 CTL epitope peptides linked via linkers, wherein the peptide having 4 linked CTL epitopes comprises the epitope peptide "PEP5" as shown in SEQ ID NO: 5 and 3 epitope peptides selected from the group consisting of the epitope peptide "PEP1" as shown in SEQ ID NO: 1, the epitope peptide "PEP2" as shown in SEQ ID NO: 2, the epitope peptide "PEP4" as shown in SEQ ID NO: 4, the epitope peptide "PEP6" as shown in SEQ ID NO: 6, the epitope peptide "PEP7" as shown in SEQ ID NO: 7, the epitope peptide "PEP8" as shown in SEQ ID NO: 8, the epitope peptide "PEP9" as shown in SEQ ID NO: 9, the epitope peptide "PEP10" as shown in SEQ ID NO: 10, the epitope peptide "PEP13" as shown in SEQ ID NO: 13, the epitope peptide "PEP15" as shown in SEQ ID NO: 15, the epitope peptide "PEP17" as shown in SEQ ID NO: 17, and the epitope peptide "PEP18" as shown in SEQ ID NO: 18 linked via linkers, wherein the peptide having 4 linked CTL epitopes may further comprise a peptide sequence consisting of hydrophilic amino acids, and wherein the peptide having 4 linked CTL epitopes has any one feature selected from the features (1) to (3) below:
(1) the peptide comprises PEP2 at the C terminus (except for the peptide comprising PEP7 and PEP8 at the N terminus successively disposed in such order from the N terminus via a linker);
(2) the peptide comprises PEP4 at the C terminus; and
(3) the peptide comprises PEP10 at the C terminus.

2. The agent for adjuvant therapy of tumors according to Claim 1, wherein the epitope peptides included in the peptide having 4 linked CTL epitopes are PEP5, PEP6, PEP9, and one epitope peptide selected from the group consisting of PEP2, PEP4, and PEP10, and wherein the C terminus of the peptide having 4 linked CTL epitopes is PEP2, PEP4, or PEP10.

3. The agent for adjuvant therapy of tumors according to Claim 1 or 2, wherein the peptide having 4 linked CTL epitopes consists of a sequence selected from the sequences indicated below:
PEP5-(L)-PEP9-(L)-PEP6-(L)-PEP4;
PEP5-(L)-PEP9-(L)-PEP6-(L)-PEP2; and
PEP5-(L)-PEP9-(L)-PEP6-(L)-PEP10,
wherein "-(L)-" represents a linker.

4. The agent for adjuvant therapy of tumors according to any one of Claims 1 to 3, wherein the peptide having 4 linked CTL epitopes consists of the sequence indicated below:
PEP5-(L)-PEP9-(L)-PEP6-(L)-PEP4,
wherein "-(L)-" represents a linker.

5. The agent for adjuvant therapy of tumors according to any one of Claims 1 to 4, wherein the linker is an amino acid linker.

6. The agent for adjuvant therapy of tumors according to Claim 5, wherein the amino acid linker is an arginine dimer or arginine trimer composed of 2 or 3 arginine residues linked to each other.

7. The agent for adjuvant therapy of tumors according to any one of Claims 1 to 6, wherein the peptide sequence consisting of hydrophilic amino acids is linked to the N terminus and is composed of an arginine trimer or arginine tetramer composed of 3 or 4 arginine residues linked to each other.

8. The agent for adjuvant therapy of tumors according to any one of Claims 1 to 7, wherein the peptide having 4 linked CTL epitopes consists of SEQ ID NO: 24, SEQ ID NO: 25, or SEQ ID NO: 26.

9. The agent for adjuvant therapy of tumors according to any one of Claims 1 to 8, wherein the peptide having 4 linked CTL epitopes consists of SEQ ID NO: 24.
